Europäisches Patentamt

European Patent Office

Office européen des brevets

(11) Veröffentlichungsnummer: **0 298 235 B1**

## (12) EUROPÄISCHE PATENTSCHRIFT

(45) Veröffentlichungstag der Patentschrift: **04.12.91**

(51) Int. Cl.<sup>5</sup>: **A61F 2/08**

(21) Anmeldenummer: **88108216.8**

(22) Anmeldetag: **21.05.88**

(54) **Zwischenwirbel-Prothese.**

(30) Priorität: **09.07.87 CH 2606/87**

(43) Veröffentlichungstag der Anmeldung:
**11.01.89 Patentblatt 89/02**

(45) Bekanntmachung des Hinweises auf die
Patenterteilung:
**04.12.91 Patentblatt 91/49**

(84) Benannte Vertragsstaaten:
**AT DE FR GB IT**

(56) Entgegenhaltungen:
**EP-A- 0 179 695**
**DE-A- 2 263 842**
**DE-A- 2 365 873**
**FR-A- 2 372 622**
**US-A- 3 867 728**

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterthur(CH)**
Erfinder: **Koch, Rudolf**
**Oberdorfstrasse 229**
**CH-8267 Berlingen(CH)**

## Beschreibung

Die Erfindung betrifft einen Zwischenwirbel-Prothese für eine Arthrodese.

Für die Versteifung zweier Lendenwirbel relativ zu einander, eine sogenannte Arthrodese, sind bisher zwei unterschiedliche Vorgehensweisen üblich. Bei dem einen Verfahren werden an anderen Stellen des Körpers, beispielsweise aus dem Becken oder dem Wadenbein, entnommene Knochensplitter als teilweiser Ersatz für die beschädigten Bandscheiben zwischen zwei Wirbel von ventral her eingesetzt und mit Hilfe von Knochenschrauben fixiert. Es hat sich gezeigt, dass - vor allem kurz nach der Implantation, wenn das Knochengewebe noch nicht mit den Wirbelkörpern verwachsen ist - die Fixierung der beiden Wirbel gegeneinander ungenügend ist, und keine ausreichende Stabilität besitzt.

Bei dem anderen Verfahren, werden zwei benachbarte Wirbel durch kreuzförmige Platten, die von dorsal mit den beiden Wirbelkörpern, beispielsweise ebenfalls durch Schrauben, fest verbunden werden, relativ zueinander fixiert. Auch hier hat die Praxis gezeigt, dass die relativ weit von den Bandscheiben entfernt angeordneten Kreuzplatten im dorsalen Bereich der Wirbel - besonders bei grösseren Bandscheibenschäden oder bei einem kompletten Ersatz einer Bandscheibe - am Ort der Bandscheiben eine ungenügende Fixierung bewirken, und ebenfalls zu Instabilitäten in der gegenseitigen Versteifung der Wirbel neigen.

Aus der DE-A-22 63 842 ist eine Bandscheibenprothese bekannt, welche mittels gewölbter Gleitflächen eine Neigung der Wirbelkörper gegeneinander gestattet.

Aus der FR-PS 76 37 174 (23 72 622) ist eine Zwischenwirbel-Prothese bekannt, die aus einer Scheibe besteht, auf deren Scheibenflächen wulstartige Erhöhungen in der Form von Kugelsegmenten vorhanden sind. Diese Scheibe wird in operativ geschaffenen, hohlkugelartigen Vertiefungen der Wirbel beweglich gelagert. Die Scheibe ist dabei steif und nicht deformierbar, während die Wulste elastisch ausgebildet sind. Eine Arthrodese zweier Wirbel ist mit dieser Konstruktion nicht möglich.

Aufgabe der Erfindung ist es, demgegenüber eine mit relativ geringem operativen Aufwand implantierbare und fixierbare Zwischenwirbel-Prothese zu schaffen, mit der als Bandscheibenersatz eine feste und stabile Versteifung zweier Wirbel erreicht wird.

Mit der vorliegenden Erfindung wird dieses Ziel dadurch erreicht, dass sie aus einem scheibenartigen Prothesenkörper besteht, der als Bandscheibenersatz von ventral her zwischen zwei Wirbel einschiebbar ist, und auf dessen Scheibenoberflächen wulstartige Erhebungen vorhanden sind, die aus einen spitzen Winkel einschliessenden Zylindersegmenten bestehen.

Die neue Prothese zeichnet sich durch grosse Einfachheit aus, da sie lediglich aus einer die Bandscheibe ersetzenden Platte besteht. Durch die wulstartigen Erhebungen, die als einen spitzen Winkel einschliessende Zylindersegmente ausgebildet sind und von ventral aus in operativ geschaffene, an die Erhebungen angepasste Bohrungen der Wirbelkörper eingelegt werden, wird die Querstabilität der Arthrodese gewährleistet und die Primärfixation sichergestellt.

Für eine Anpassung der Prothese an die anatomischen Gegebenheiten ist es zweckmässig, wenn der prothesenkörper nierenförmig ausgebildet ist, und/oder wenn die Dicke des Prothesenkörpers sich von ventral nach dorsal konisch verjüngt. Weiterhin ist es vorteilhaft, wenn der von Erhebungen eingeschlossene Winkel $\pi/2$ beträgt.

Insbesondere haben sich Ausführungsformen bewährt, bei denen die Scheibe ein Kunststoff, z.B. Polyethylen der für Implantate üblichen Spezifikationen, ist, und ihre Flächen mit einem Metallgitter, beispielsweise einem mehrlagigem Drahtnetz aus Titan oder einer Titanlegierung belegt sind, um das Ein- und Anwachsen von Gewebe und damit die Langzeitfixierung zu fördern. Bei einer zweiten besonders geeigneten Ausführungform, besteht die Scheibe selbst aus Titan oder einer Titanlegierung; ihre Scheibenflächen sind ebenfalls mit einer das Ein- oder Anwachsen von Gewebe fördernden Struktur versehen, die beispielsweise gleichfalls durch ein Drahtnetz gebildet sein kann, das mit über die Flächen verteilten Punktschweissungen auf der Scheibe fixiert ist.

Die Primärfixation der Prothese bis zum Einwachsen von Gewebe, kann erheblich durch mit Durchtrittsöffnungen für Knochenschrauben versehene Laschen am konvexen, ventralen Rand des Prothesenkörpers verbessert werden. Diese Laschen können sowohl in den Prothesenkörper integriert, als auch als zusätzliche Einzelelemente vorhanden sein; sie werden in die Wirbelkörper der beiden Wirbel eingeschraubt, wobei diese zuvor durch Zusammenpressen auf Druck vorgespannt sein können.

Im folgenden wird die Erfindung anhand von Ausführungsbeispielen im Zusammenhang mit der Zeichnung näher erläutert.

Fig. 1    ist eine Aufsicht auf eine erste Ausführungsform der neuen Prothese;

Fig. 2    gibt den Schnitt II-II von Fig. 1 wieder;

Fig. 3    zeigt in gleicher Darstellung wie Fig. 1 eine weitere Ausführungsform der Erfindung;

Fig. 4     ist eine Ansicht von Fig. 3 von oben;

Fig. 5 und 6     geben zwei verschiedene Ausführungsformen für die in die Wirbel einschraubbaren Laschen zur primären Fixierung der Prothese wieder.

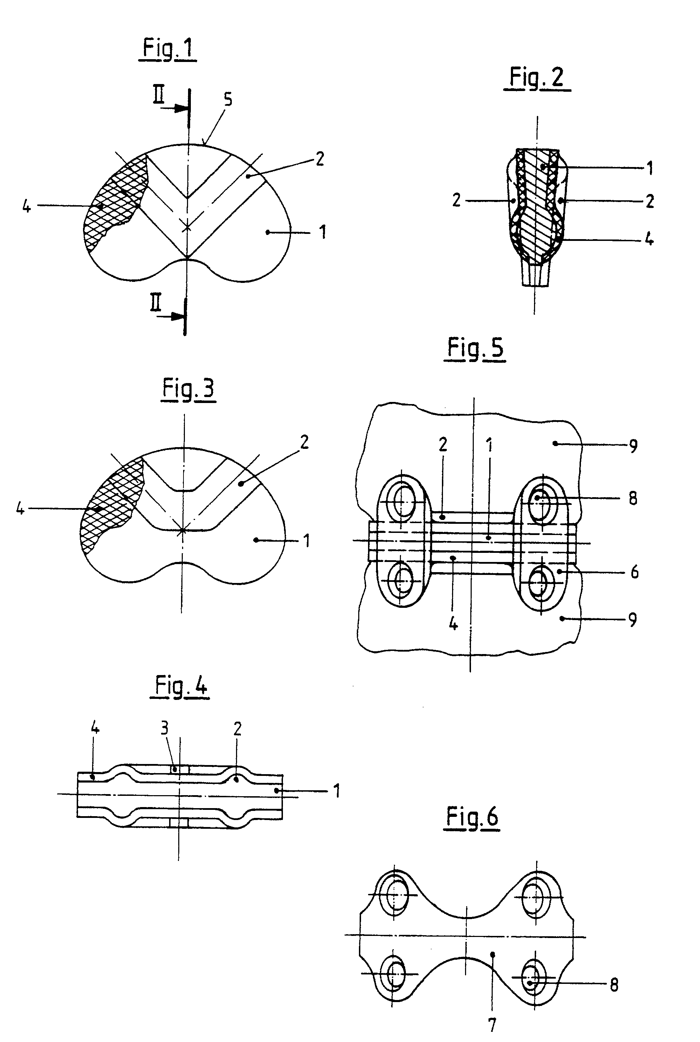

Die den Prothesenkörper bildende Scheibe 1, die nieren-oder bohnenfömig ausgebildet ist, wobei die konvexe Krümmung nach ventral gerichtet ist, hat eine von ventral nach dorsal abnehmende Dikke (Fig. 2). Die Scheibe 1 ist zur Fixierung in den Wirbelkörpern auf ihre Scheibenflächen mit wulstartigen Erhebungen 2 versehen, die die Form von Zylindersegmenten haben. Die beiden Zylindersegmente stehen senkrecht aufeinander, ihrer Achsen schliessen also einen Winkel von $\pi/2$ ein. Bei dem ersten Beispiel (Fig. 1) reichen die Erhebungen 2 bis zum dorsalen Rand, während sie bei der zweiten Ausführungsform (Fig. 3) im Bereich des Winkelscheitels abgeschnitten und durch einen brückenartigen Steg 3 ersetzt sind.

Beide Scheibenflächen, einschliesslich der Erhebungen 2, sind mit einem mehrlagigen Titan-Drahtnetz 4 belegt, dass in bekannter Weise mit mindestens einer Lage in den Kunststoff der Scheibe 1 eingebettet ist. In bekannter Weise, dient die Netzauflage als das An- und Einwachsen von Gewebe fördernde Struktur.

Wie Fig. 5 zeigt, kann für eine Verbesserung der Primärfixierung - bevor an bzw. in das Implantat Knochengewebe an- bzw. eingewachsen ist - der ventrale Rand 5 (Fig. 1) des Prothesenkörpers 1 von Laschen 6 abgedeckt sein. Während in Fig. 5 zwei getrennte Laschen 6, die den Rand 5 nur teilweise abdecken gezeigt sind, stellt Fig. 6 eine als Kreuzplatte 7 ausgebildete Lasche dar, die den ventralen Rand 5 des Prothesenkörpers 1 auf seinen ganzen Umfang abdeckt. Die Laschen 6 oder 7, können dabei sowohl ein integraler Bestandteil des Prothesenkörpers 1 sein als auch getrennte Einzelelemente vorliegen. Sie weisen Durchtrittsöffnungen 8 für Knochenschrauben auf, die in die Wirbelkörper 9 eingeschraubt werden.

Die Oeffnungen 8 sind als Teil einer Kugelschale ausgeführt, um eine Ausrichtung der nicht gezeigten Schrauben in eine beliebige Richtung zu erleichtern.

Wie bereits angedeutet, werden die Laschen 6 bzw. 7 in die Wirbelkörper eingeschraubt, nachdem diese zuvor leicht zusammengepresst worden sind, um so einen Anpressdruck der Wirbel 9 auf dem Prothesenkörper 1 zu erzeugen.

## Patentansprüche

1. Zwischenwirbel-Prothese für eine Arthrodese, die aus einem scheibenartigen Prothesenkörper (1) besteht, der als Bandscheibenersatz von ventral her zwischen zwei Wirbel (9) einschiebbar ist, und auf dessen Scheibenoberflächen wulstartige Erhebungen (2) vorhanden sind, dadurch gekennzeichnet, daß die wulstartige Erhebungen aus einen spitzen Winkel einschliessenden Zylindersegmenten bestehen.

2. Zwischenwirbel-Prothese nach Anspruch 1, dadurch gekennzeichnet, dass der Prothesenkörper (1) nierenförmig ausgebildet ist.

3. Zwischenwirbel-Prothese nach Anspruch 1, dadurch gekennzeichnet, dass die Dicke des Prothesenkörpers (1) sich von ventral nach dorsal konisch verjüngt.

4. Zwischenwirbel-Prothese nach Anspruch 1, dadurch gekennzeichnet, dass der von den Erhebungen (2) eingeschlossene Winkel $\pi/2$ beträgt.

5. Zwischenwirbel-Prothese nach Anspruch 1, dadurch gekennzeichnet, dass der Prothesenkörper (1) aus Kunststoff besteht, und seine Oberflächen mit einem Metallgitter (4) belegt sind.

6. Zwischenwirbel-Prothese nach Anspruch 1, dadurch gekennzeichnet, dass der Prothesenkörper (1) aus Titan oder einer Titanlegierung besteht, und dass seine Oberflächen mit einer dass Anwachsen von Gewebe fördernden Struktur versehen sind.

7. Zwischenwirbel-Prothese nach Anspruch 1, dadurch gekennzeichnet, dass der ventrale Rand (5) des Prothesenkörpers (1) mindestens teilweise durch mit Durchtrittsöffnungen (8) für Knochenschrauben versehen Laschen (6, 7) abgedeckt ist.

## Claims

1. An intervertebral prosthesis for an arthrodesis in the form of a discoid prosthesis body (1) introducible as an intervertebral disc replacement from the ventral direction between two vertebrae (9), bead-like protuberances (2) being present on its disc surfaces, characterised in that the bead-like protuberances take the form of cylinder segments which include an acute angle.

2. A prosthesis according to claim 1, characterised in that the prosthesis body (1) is reniform.

3. A prosthesis according to claim 1, characterised in that the thickness of the prosthesis body (1) narrows conically ventrally to dorsally.

4. A prosthesis according to claim 1, characterised in that the angle included by the protuberances (2) is $\pi/2$.

5. A prosthesis according to claim 1, characterised in that the prosthesis body (1) is made of plastics and its surfaces are covered by a metal mesh (4).

6. A prosthesis according to claim 1, characterised in that the prosthesis body (1) is made of titanium or a titanium alloy and its surfaces have a structure promoting the ongrowth of tissue.

7. A prosthesis according to claim 1, characterised in that the ventral edge (5) of the prosthesis body (1) is covered at least to some extent by tabs (6, 7) formed with apertures (8) for bone screws.

**Revendications**

1. Prothèse de disque intervertébral pour une arthrodèse, qui est constituée d'un corps de prothèse (1) en forme de disque qui est inséré, à partir du côté ventral, entre deux vertèbres (9), en remplacement d'un disque intervertébral, et qui présente sur ses surfaces de disque des reliefs (2) en forme de bourrelets, caractérisée en ce que les reliefs en forme de bourrelets sont constitués de segments cylindriques formant entre eux un angle aigu.

2. Prothèse de disque intervertébral selon la revendication 1, caractérisée en ce que le corps de prothèse (1) est réniforme.

3. Prothèse de disque intervertébral selon la revendication 1, caractérisée en ce que l'épaisseur du corps de prothèse (1) se rétrécit coniquement du côté ventral vers le côté dorsal.

4. Prothèse de disque intervertébral selon la revendication 1, caractérisée en ce que l'angle formé par les reliefs (2) est égal à $\pi/2$.

5. Prothèse de disque intervertébral selon la revendication 1, caractérisée en ce que le corps de prothèse (1) est en matière plastique et ses surfaces sont recouvertes d'une grille métallique (4).

6. Prothèse de disque intervertébral selon la revendication 1, caractérisée en ce que le corps de prothèse (1) est en titane ou dans un alliage de titane et en ce que ses surfaces sont pourvues d'une structure favorisant l'adhérence du tissu.

7. Prothèse de disque intervertébral selon la revendication 1, caractérisée en ce que le bord ventral (5) du corps de prothèse (1) est recouvert au moins partiellement par des éclisses (6, 7) pourvues d'ouvertures de passage (8) pour les vis à os.

Fig.1

Fig.2

Fig.3

Fig.4

Fig.5

Fig.6